# EUROPEAN PATENT APPLICATION

(11) **EP 4 825 199 A1**
(43) Date of publication of application: **21.10.2026**
(21) Application number: 25214771.5
(22) Date of filing: 10.06.2020
(51) Int. Cl.: A24F 40/10, A24F 40/51, A24F 40/70, A24F 40/485

(54) **DISPOSABLE VAPORIZER**

(30) Priority: 10.06.2019 US 201962859439 P
(62) Document number(s) of the earlier application(s) in accordance with Art. 76 EPC: 25186811.3; 20823452.6 / 3 980 100
(71) Applicant: SkyX IP Holdings I LLC, New York, NY 10001 (US)
(72) Inventor: RYAN, Andrew, Boston, 02115 (US); STEINBAUER, Martin, New York, 10036 (US)
(74) Representative: CH Kilger Anwaltspartnerschaft mbB

(57) **Abstract**

A vaporizer device including: 1) a body having a mouthpiece and an interior chamber, 2) a reservoir inserted into the interior chamber of the body for storing an aerosolizable material, 3) a housing inserted into the interior chamber including a heating chamber including a heating element and a pressure sensor for controlling operation of the heating element, 4) a power source for powering the heating element and pressure sensor, and 5) a vapor tube extending from the mouthpiece to the heating chamber. The vapor tube is integrally formed with the reservoir, or with the body. The device may in addition also include a PCB tube extending from the mouthpiece to the pressure sensor, which is integrally formed with one of the reservoir or the body.

## Description

### FIELD OF THE INVENTION

This disclosure relates generally to disposable vaporizers, and more particularly, to disposable vaporizers for e-liquids having leak-proof features.

### BACKGROUND

Disposable vaporizers for delivering a vaping experience based on e-liquids are presently in the marketplace. These devices present a number of challenges for their providers, for example including:
1. Leakage
   a. Liquid leaking out of the fluid container either through the vapor tube at the top, or at the bottom at the air holes of a device.
2. Reliability
   a. A tube that acts as both a vapor tube and an air flow activation tube may, due to minor leakage, delay activation of device, or change the intended airflow of the device during use.
3. Fill-volume/space optimization
   a. Due to the desirable small and ergonomic designs of disposable vaporizers, internal space is limited. It is desirable to keep the device ergonomic while fitting a stronger battery and increased the fill-volume for liquids.
4. Flavor and nicotine/other chemical delivery
   a. Vaporizers where liquid sits in a cotton medium, do not optimally generate aerosols with flavor or nicotine representative of the same liquid in other devices. This is likely because the cotton absorbs elements of the flavoring and/or nicotine, thus these chemicals stay behind and are not being passed through to the heating element and consequently to the aerosol.
5. Simple filling and sealing of device
   a. Current constructions rely on complicated mechanisms and assembly steps to fill the liquid into the liquid container and then sealing the filled liquid container. In particular, this filling is often done manually. These steps would be difficult to automate. Many reasons exist why filling should be done as simply as possible, to also provide the opportunity to fill in other countries or areas. None of these constructions are optimized for simple filling.
6. Inspect fill level of disposable device
   a. Users may want to inspect the fill level of the disposable device to see how much of the liquid they purchased or have left.
7. Safety and protection of minors
   a. Currently users may through minor force open the press-fit caps at the bottom of the device, and extract the battery and the fluid container. This may pose risks to adult and or minor users if they come into contact with either battery or the fluid held in the container.

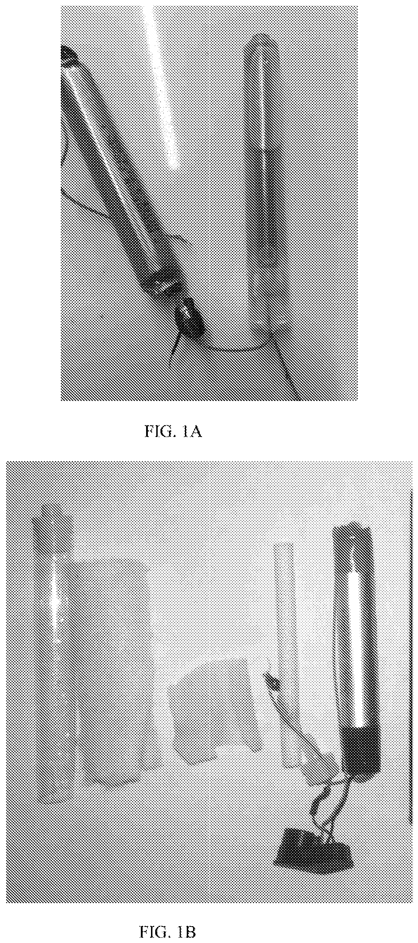

Two types of constructions currently on the market for disposable one-piece vaporizers with e-liquids are prevalent:
1. "Oil Cup" - Internal constructions with round containers containing liquid, and on the inside a tube centrally running through the container, such that the liquid sits in between the tube and the outer shell of the container. The liquid is wicked by a cotton/silica wick placed inside a cup, into the cup, whereas the cup is placed underneath the vapor tube. A heating element generally made from stainless steel, kanthal, nichrome, or the like, is wrapped around the wick, and the activation of the disposable device happening through either a button or a pressure sensor that detects a change in pressure as the user inhales. The heating wire is then heated and vaporizes the liquid, the aerosol generated travels through the vapor tube and exits the disposable device towards the mouthpiece. An example of this type of construction is illustrated in FIG. 1A.
2. "Cotton Medium and Wick/Coil Through Vapor Tube" - These vaporizers have internal constructions with round containers containing e-liquid. Centrally inside of the container a tube is placed, the tube either being manufactured from metal or a fabric material. Along the tube, either a cut or an inlet is created to place a coil and a wick. The coil and the wick are placed such that the two ends of the cotton are ex-posed to the two openings and in contact with the liquid.

In between the tube and the exterior of the liquid container, cotton is placed and liquid is filled into the space with the cotton. Thus, the liquid is sus-pended in the cotton, and both cotton and liquid form a reservoir. The cotton acts as a storage medium, but also as a wicking element to wick the liquid to the wick placed inside/through the vapor tube. Additionally, cotton wraps may be added to further seal or wick liquid towards the heating element. An example of this type of construction is illustrated in FIG. 1B.

It would be beneficial to develop a vaporizer cartridge that addresses the above-identified challenges.

### SUMMARY

By way of example, aspects of the present disclosure are directed to disclose a novel vaporizer device. In accordance with aspects of the present disclosure, the disclosed vaporizer device includes: 1) a body having a mouthpiece and an interior chamber, 2) a reservoir inserted into the interior chamber of the body for storing an aerosolizable material, 3) a housing inserted into the interior chamber including a heating chamber including a heating element and a pressure sensor for controlling operation of the heating element, 4) a power source for powering the heating element and pressure sensor, 5) a vapor tube extending from the mouthpiece to the heating chamber, and 6) a PCB tube extending from the mouthpiece to the pressure sensor. Significantly, the vapor tube and PCB tube are integrally formed with the reservoir.

In accordance with an additional aspect of the disclosure, the aerosolizable material is an e-liquid.

In accordance with an additional aspect of the disclosure, the vapor tube and the PCB tube are formed as a single component.

In accordance with an additional aspect of the disclosure, each of the vapor tube and the PCB tube comprise a metal hypotube.

In accordance with an additional aspect of the disclosure, the mouthpiece is positioned at a top end of the body, the housing positioned near a lower end of the body, the reservoir is positioned intermediate the mouthpiece and the power source, and the vaporizer device further comprises a reservoir seal engaging an interior surface of the body.

In accordance with an additional aspect of the disclosure, the vaporizer device further comprises an end cap inserted into the interior chamber below the housing, which interferingly and securely fits into the housing to retain the housing and the reservoir within the body.

In accordance with an additional aspect of the disclosure, the vaporizer device includes at least one air vent in fluid communication with the vapor tube and an exterior surface of the body.

In accordance with an additional aspect of the disclosure, the vaporizer device includes at least one of a light or a vibration motor for indicating one or more of an activation status, activation time or dosage.

In accordance with an additional aspect of the disclosure, the vapor tube and PCB tube are alternatively positioned along opposing interior surfaces of the body.

In accordance with an additional aspect of the disclosure, the vapor tube and PCB tube alternatively are integrally formed with the body.

This SUMMARY is provided to briefly identify some aspects of the present disclosure that are further described below in the DESCRIPTION. This SUMMARY is not intended to identify key or essential features of the present disclosure nor is it intended to limit the scope of any claims.

### BRIEF DESCRIPTION OF THE DRAWING

A more complete understanding of the present disclosure may be realized by reference to the accompanying drawing in which:
FIG. 1A illustrates components of a first type of prior art inhalable vaporizer device;
FIG. 1B illustrates components of a second type of prior art inhalable vaporizer device;
FIG. 2A provides an external view of a vaporizer device according to aspects of the present disclosure;
FIG. 2B illustrates internal elements of the device of FIG. 2A.
FIG. 2C depicts battery technologies that may be used in inhalable vaporizer devices;
FIG. 2D illustrates a first type of molded reservoir and vapor/PCB tube component for use in a device according to aspects of the present disclosure;
FIGs. 3A, 3B and 4 illustrate a second type of molded reservoir and vapor/PCB tube component of a device according to aspects of the present disclosure;
FIG. 5 illustrates a sealing element for a device in accordance with aspects of the present disclosure;
FIG. 6 illustrates a PCB and wick housing for a device in accordance with aspects of the present disclosure;
FIGs. 7 and 8 illustrates a heating coil assembly for a device in accordance with aspects of the present disclosure;
FIGs. 9 and 10 further illustrate the heating coil assembly of FIGs. 7 and 8;
FIG. 11 provides a cross-sectional view of the heating coil assembly of FIGs. 7 and 8;
FIG. 12 illustrates another sealing element for a device in accordance with aspects of the present disclosure;
FIGs. 13 and 14 further a wick and PCB housing for a device in accordance with aspects of the present disclosure;
FIGs. 15A and 15B illustrate a method for assembling a device in accordance with aspects of the present disclosure;
FIGs. 15C and 15D illustrate another type of molded reservoir and vapor/PCB tube component for use in a device according to aspects of the present disclosure
FIGs. 16A and16B illustrate sealing elements for a device according to aspects of the present disclosure;
FIGs. 17A - 19B illustrate fill window designs for a device according to aspects of the present disclosure;
FIGs.20A, 20B and 21 illustrate a ceramic heating element application for a device according to aspects of the present disclosure;
FIGs. 22A and 22B illustrate an alternate tube and cavity design for a device according to aspects of the present disclosure;
FIGs. 23A and 23B illustrate an alternate outer shell design with inner partitioning for a device according to aspects of the present disclosure;
FIGs. 24A and 24B illustrate another alternate outer shell design for a device according to aspects of the present disclosure
FIGs. 25A and 25B illustrate another housing design configurations for a device according to the present disclosure;
FIG. 26 illustrates a mouthpiece design for a device according to the present disclosure;
FIG. 27 illustrates an outer shell design for the device of FIG. 26;
FIGs. 28A - 29C illustrate outer shell designs for a device according to the present disclosure;
FIGs. 30A, 30B and 31 illustrate an additional outer shell design for a device according to the present disclosure;
FIGs. 32 and 33 illustrate a battery cavity design for a device according to aspects of the present disclosure;
FIGs. 34A - 34D illustrate an additional outer shell design for a device according to the present disclosure;
FIGs. 35A - 35C and 36A - 36B illustrate another outer shell design for a device according to the present disclosure;
FIGs. 37A and 37B illustrate an additional outer shell design for a device according to the present disclosure; and
FIG. 38 illustrates a baseplate design for a device according to aspects of the present disclosure.

### DETAILED DESCRIPTION

The following merely illustrates the principles of the disclosure. It will thus be appreciated that those skilled in the art will be able to devise various arrangements which, although not explicitly described or shown herein, embody the principles of the disclosure and are included within its spirit and scope.

Furthermore, all examples and conditional language recited herein are principally intended expressly to be only for pedagogical purposes to aid the reader in understanding the principles of the disclosure and the concepts contributed by the inventor(s) to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements later developed that perform the same function, regardless of structure.

Unless otherwise explicitly specified herein, the drawings are not drawn to scale.

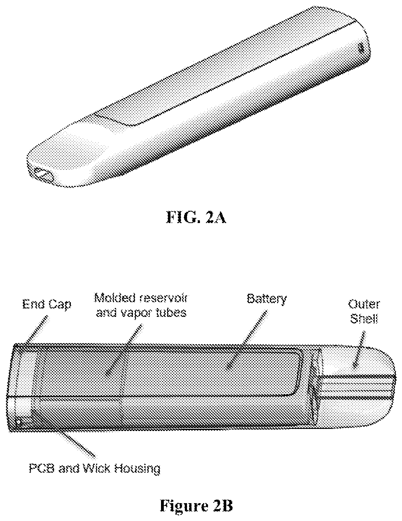

Aspects of the present disclosure are directed to a novel vaporizer-based inhalation device. An exemplary device is illustrated in FIG. 2A. From the outside, the device has one continuous shell. The raised surface on the body of the device may be designed, for example, to have a matte finish with the rest of the plastic having a gloss finish. In operation, the user simply puts their lips around the mouthpiece and inhales in to activate the device through a pressure sensor.

Internal elements of the device are illustrated in FIG. 2B. To help reduce cost and increase fluid volume, the reservoir and vapor/PCB tubes may preferably be formed as a single molded component. "PCB" stands for a Printed Circuit Board, and in this construction, may include a pressure sensor that detects the change in pressure upon a user's inhalation which triggers the activation of the electric current to the heating element and begins the vaporization process. The PCB's function includes but is not limited to regulating the power supply delivery to the heating element, be coupled to a lighting mechanism indicating activation, and may be connected to a vibration motor for tactile feedback when a certain time of inhalation or corresponding dose of a material is generated in an aerosol and inhaled by the user. This disclosure refers to a "PCB tube" as a tube that connects from the inhalation points to the PCB chamber and acts as an activator of the device. In contrast, the vapor tube acts as the tube carrying the aerosol to a user. It is advantageous to keep the PCB tubes and vapor tubes separate, as these serve separate functions, but also, minor leakage may affect the PCB adversely. If minor leakage were to cover the PCB, the PCB's pressure sensor may not activate upon a user's inhalation, rendering the device inoperable. However, embodiments of this design may also simply include a single tube that functions as both the airflow activation and the vapor tube if sealing and molded parts are tightly controlled, though an element of risk remains. The reservoir is closed off fluidically with the PCB and Wick housing with the entire assembly locked into the outer shell with an end cap.

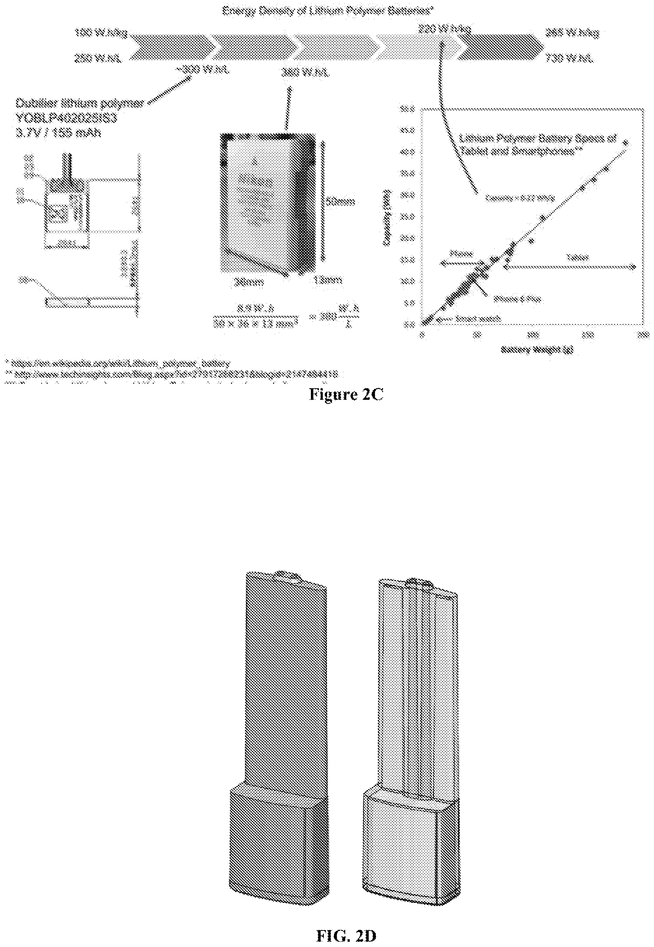

FIG. 2C illustrates a variety of battery technologies with different energy densities that may be usable in a device as described in this disclosure. Most batteries found in vaporizer devices are on the lower end of the energy density spectrum and often around 250 Wh/L, which means that for the space they occupy, they provide little power. Current batteries take up too much space, and smaller batteries with low energy density will likely be more expensive to produce and source. Thus, it is desired to use higher energy density batteries and also make the batteries smaller. Preferably, for the device described by the present disclosure, the battery to be used is 15mm wide, 4mm deep, and 36mm long Li-Mn battery. It has a capacity of 400 MaH leading to an energy density of approximately 500 Wh/L.

FIG. 2D further illustrates a molded reservoir and vapor/PCB tube component of a device according to aspects of the present disclosure. The vapor tubes travel along the entire length of the back spine of this part from the mouthpiece to the bottom of the reservoir. The open space alongside of these tubes in addition to the main cavity towards the bottom acts as the volume for the oil. The current design may preferably approximately 1.63mL of oil. However, increasing the overall length by 5mm could allow a 2mL fill-volume. Conversely, decreasing the fill volume is possible by decreasing the height of the inserted reservoir, for instance, by needing space for additional hardware components such as a vibration motor, or to add more and/or stronger seals. In certain circumstances, a lower fill level may also be desired commercially if a commercially expensive oil (e.g. cannabis oils) are filled into the device. Embodiments of this design may take into account a variety of battery shapes (e.g. two smaller round shapes fit on the side of the reservoir) or a variety of outer shell aesthetic shapes (e.g. rounder, or rectangular, smaller, larger, thicker, thinner).

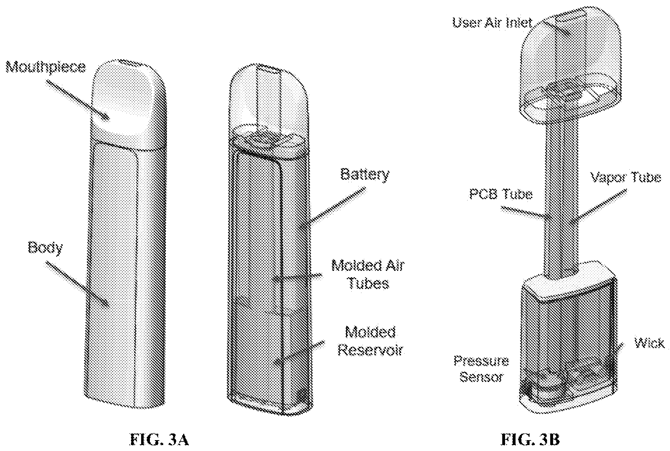

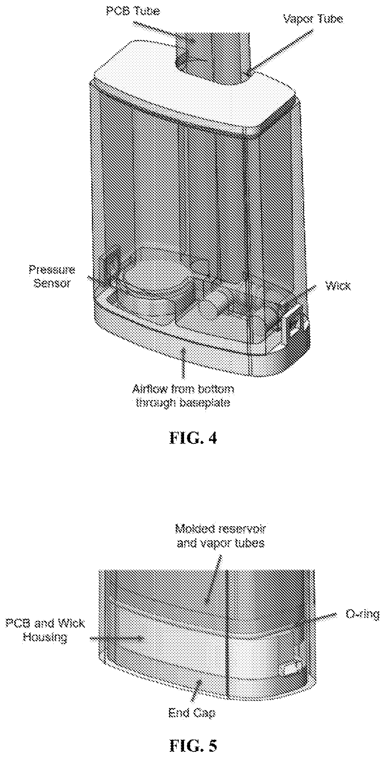

FIGs. 3A, 3B and 4 illustrate a second type of molded reservoir and vapor/pcb tube component of a device according to aspects of the present disclosure, alone and as positioned in the outer shell. In this component, the space beside the tubes is not used for liquid storage. This embodiment incorporates the molded air cavities into the molded reservoir. As in the first embodiment type described above, the air cavities are directly incorporated into the reservoir to help keep a solid seal between the fluid to vapor path. However, unlike the construction above, the pod is not molded in one, but the pod has a cap (as illustrated in FIG. 4). Any embodiments that have more or separate parts, but satisfy the general construction, would be substantially equivalent and contemplated by the disclosure herein. The device internals to be fully assembled, with the last step being to encase them in the outer shell.

For the examples described above, the bottom of this inserted pod is open. FIG. 5 shows how the PCB and Wick housing compresses against an O-ring to create a face seal and seal off the bottom of the reservoir. Additionally, as the O-ring is compressed, it pushes against the inner walls of the shell to further seal the device. The PCB and Wick housing has 2 ports where oil can flow to get in contact with the wicks. In addition to sealing the reservoir fluidically, there is also a face seal to ensure that the vapor and PCB tubes are sealed to their specific compartments.

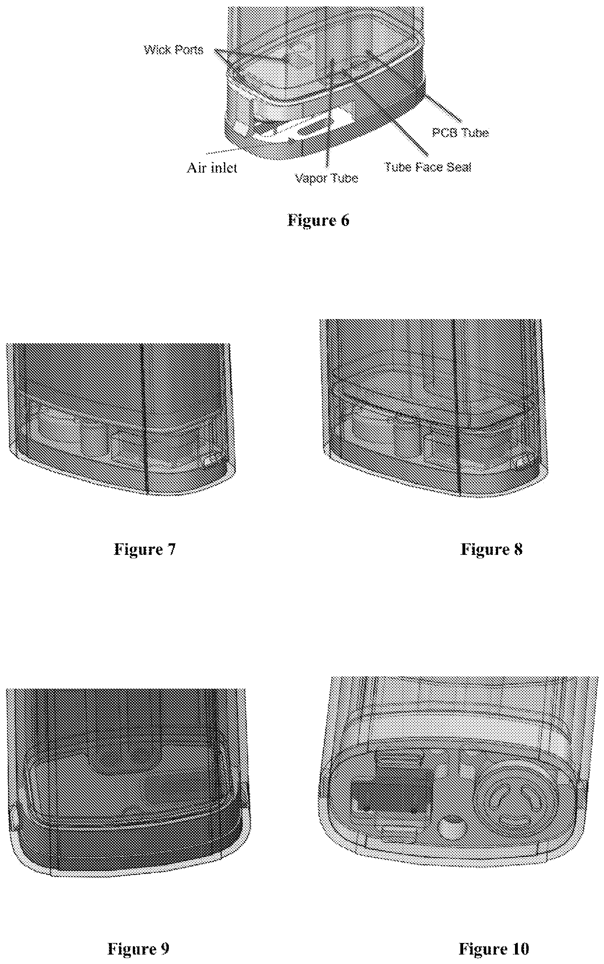

Other anti-leaking mechanisms are in place in addition to the O-ring and the Tube Face Seal described above. In the embodiments shown in FIG. 5 with a cotton wick and a metal heating coil and FIGs. 6 - 11 with ceramic, the sealing as well as the size, diameter and wicking capabilities of the wick can be optimized to reduce the risk of leaking into the chamber that is directly connected to the vapor tube. This chamber may sometimes be referred to as the heating chamber. In addition, the heating element is placed on an area with a cavity or depressed area. This cavity, as seen for example in FIG. 6 to the left of the air inlet slot, can act as a reservoir to hold excess liquid should it leak from the wick. In addition, the following leaking prevention mechanisms may also be employed with the current construction. Cotton may be added either directly under the heating coil, beside the vapor tube, or beside the air inlet. Cotton can absorb excess liquid in addition to or instead of the leaking cavity. Another possibility would be to add a taller wall in between the air inlet and the wick/coil, which would prevent excess liquid from seeping through the air inlet or through the vapor tube.

Furthermore, the sealing and fluid flow may also be adapted for ceramic coil materials as described in the following paragraphs, and an example illustrated in FIGs. 6 - 11. In the construction above, the wick ports funnel liquid to the wick, and the wick and coil construction vaporize liquids into aerosols. With a ceramic heating element, the fluid flow would still include one or two liquid ports, but sealing would be adapted (including for example but not limited to a top seal, an o-ring between the top seal and the ceramic heating element, and a holding structure for the ceramic coil) and the sealing/connection to the vapor tube would also be optimized for this configuration.

Oil from the reservoir falls into the cup of the ceramic coil. The coil is illustrated for example in FIGs. 9 and 10. Only the top face of the coil where the cup is located is open to the reservoir. The oil is absorbed by the ceramic, and the coil becomes saturated. When the device is activated, the coil heats up causing the saturated oil to vaporize. The lower half of the 4 outer walls of the cup and the bottom face of the ceramic coil are open to air. When the device is activated, air is pulled across the coil pulling the vaporized oil up and into the vapor tubes towards the mouthpiece of the device.

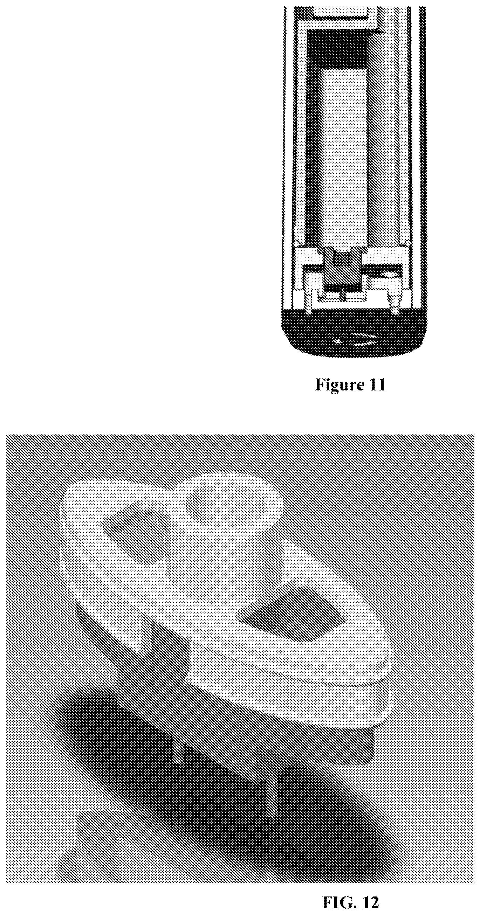

FIGs. 7 and 8 show a side view illustrating how the ceramic coil can be fit into the base plate; one of the two air inlets is shown at the front of the figures. In the non-ceramic embodiments, one air inlet may preferably be used. In a ceramic embodiment, either one or two air inlets may preferably be used. Air inlets are the points where air can enter the heating chamber from outside the device, and mix with the generated aerosol, before it travels through the vapor tube to the user. It is described in more detail later. By way of example, a two air inlet ceramic embodiment is illustrated by FIGs. 9 and 10. FIG. 10 shows a bottom view of how the ceramic coil is fit into the device. FIG. 11 shows how the ceramic coil is fit from a cross sectional view, right above an area demarcated by raised walls, where excess fluid or condensation can be stored.

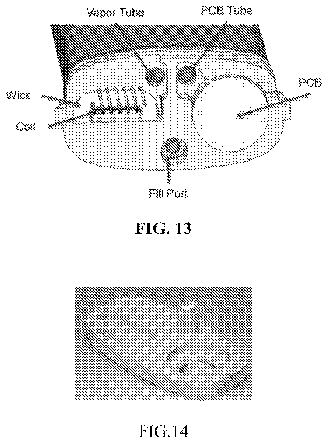

FIG. 12 shows a second embodiment of how liquid can be funneled into the ceramic heating element; in this embodiment, the ceramic coil would be placed underneath the vapor tube. Vapor generated at the top of the ceramic heating element, as well as vapor from the sides, can then travel through the vapor tube. These aspects are further illustrated by the elements of the wick and PCB housing shown in FIG. 13.

For the Wick/Coil embodiments, once the fluid is in contact with the wick, it is drawn along the wick to the coil. When the user inhales on the mouthpiece air flows through both the vapor and the PCB tubes. A pressure sensor on the PCB detects the difference in pressure and triggers the device to allow power to the coil causing it to heat up. This heat causes the oil in the wick to evaporate. This evaporated liquid is drawn up the Vapor tube to the user. FIG. 13 also shows the Fill Port. This is where the reservoir is filled from. Once the fluid is injected into the reservoir from this port, part of the end cap comes in to plug it and seal it off completely. It is important to note that while a vapor and PCB tube have advantages of separating the function for these tubes, and addressing problems such as leakage affecting the airflow, a further embodiment may use a joint (single) air tube for this purpose. The fill port is used for filling liquid into the liquid reservoir. Upon completed filling, the bottom cap with the stopper is then inserted into the fill port, and the device would be sealed.

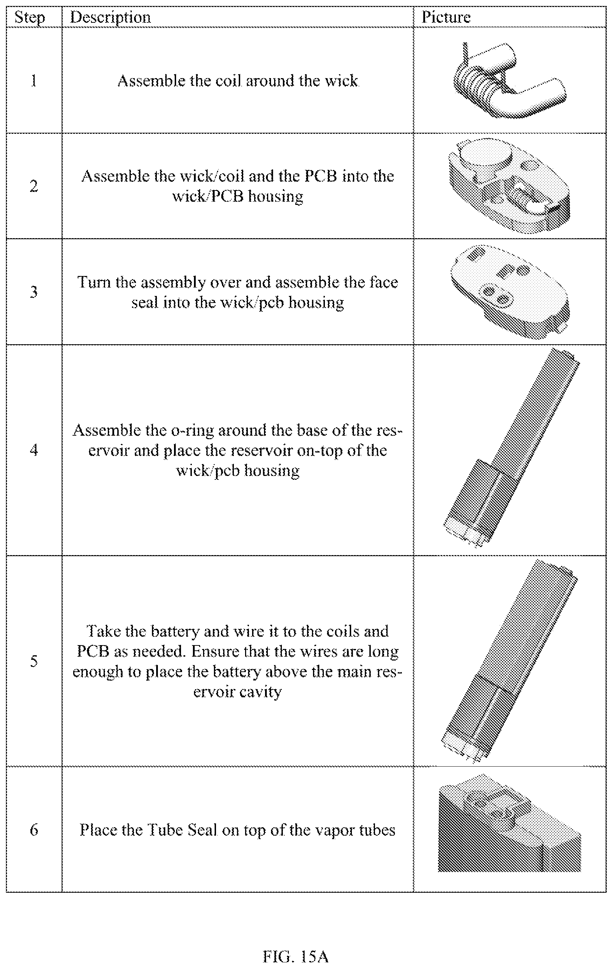

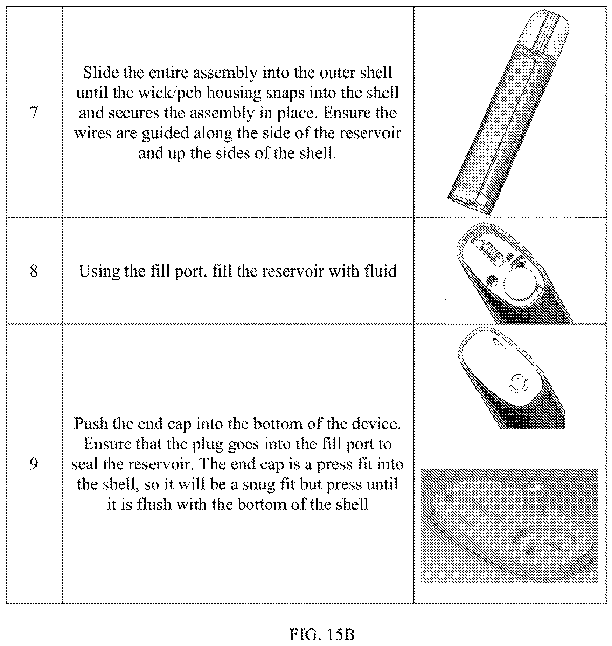

An exemplary assembly process for the vaporizer as described in shown in FIGs. 15A and 15B.

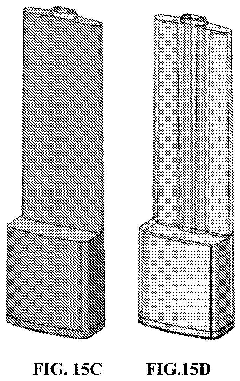

A number of alternate design constructions are contemplated by the present disclosure. For the molded reservoir and vapor tubes embodiment as illustrated by FIGs. 15C and 15D, a risk is the long thin tubes that need to be molded. This is a long draw for such a small cavity. Alternatively, the tubes may be formed for example by insert molding twin metal hypo-tubes.

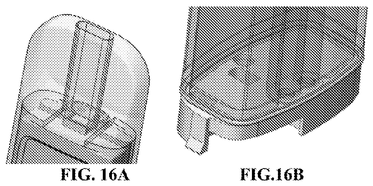

To maintain the outer form factor and to maximize the available fluid volume, most fluidic seals are preferably face seals. If the seals do not have enough compression, then they will leak. The compression comes from the wick/pcb housing snapping into the outer shell. To get to the point where it snaps in, the seals must compress and in turn they make a fluidic seal. However, if parts are out of spec, it is possible that there won't be enough compression which in turn causes the device to leak. Sealing optimizations may be performed during the manufacturing process such as adding additional seals or modifying the existing ones. The seal at the top of the device near the mouthpiece and the two seals at the bottom of the vapor/air tubes (as respectively illustrated for example in FIGs. 16A and 16B) are examples of the face seals described in the paragraph above. They rely on compression to seal against the 2 bodies they sit between.

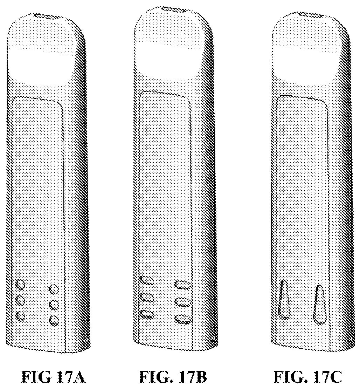

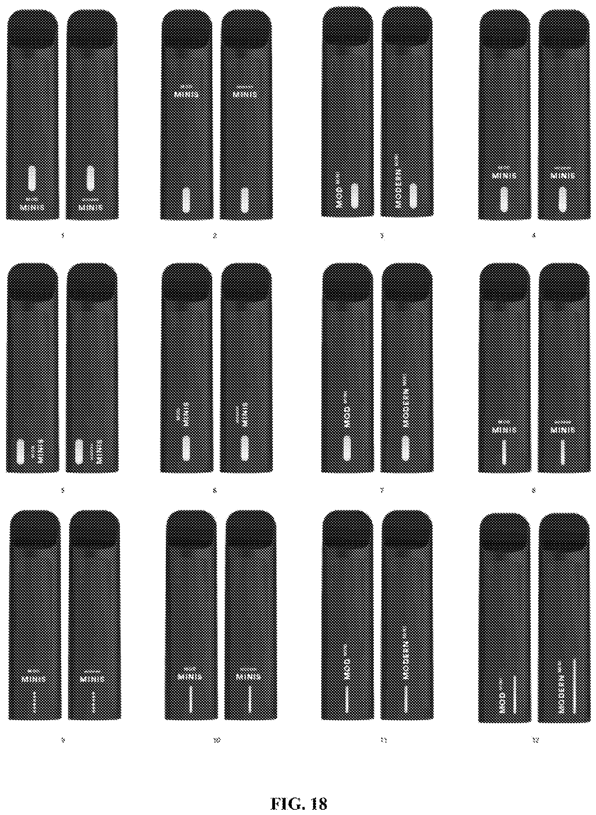

The following section further describes the ceramic heating coil embodiment as described earlier. As the ceramic coil is often used for higher viscosity liquids, and these liquids tend to be of higher commercial value and more expensive to the user, the user may want to track the fill level of the liquid as it is used. Further, a fill level will allow a user to track if the device was full at the point of purchase. These windows may be placed on either face of the device at any position (top, middle, bottom), or also only on one face of the device as illustrated for example in FIGs. 17A - 17C. Natural light is effectively present as backlighting in the two face embodiments for improved fill level visibility. Alternatively or additionally, windows may be placed on the sides of the devices where the snaps reside, or may be created such that the snaps are integrated into the window. In other words, the snap on the side could push past the beginning of a window on a side, and then snap would extend out at the beginning of the window, effectively securing the insertable part from the bottom. These windows can be referred to as fill-level indication windows as well. Fill-level windows may further be used with other heating elements than ceramic coils, such as a traditional cotton/silica and kanthal, nichrome, or stainless steel. A variety of embodiments are shown below in FIGs. 17A - 19B.

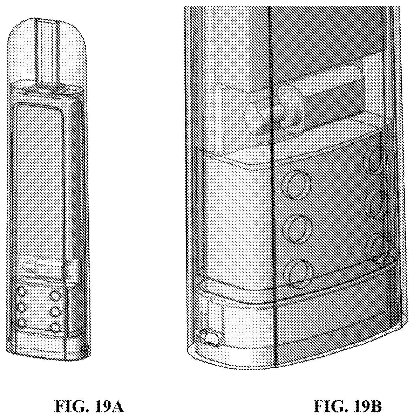

The fill level window embodiment illustrated in FIGs. 19A and 19B also shows a vibration motor positioned on a PCB adjacent to a shelf surface of the reservoir. The vibration motor may be used for example to indicate to a user when a specific inhalation time has been reached or when a specific dose associated with the inhalation time, temperature, and material is reached. Other embodiments of this dosing indication may be achieved for example by sound or lighting features, in addition or instead of a vibration motor.

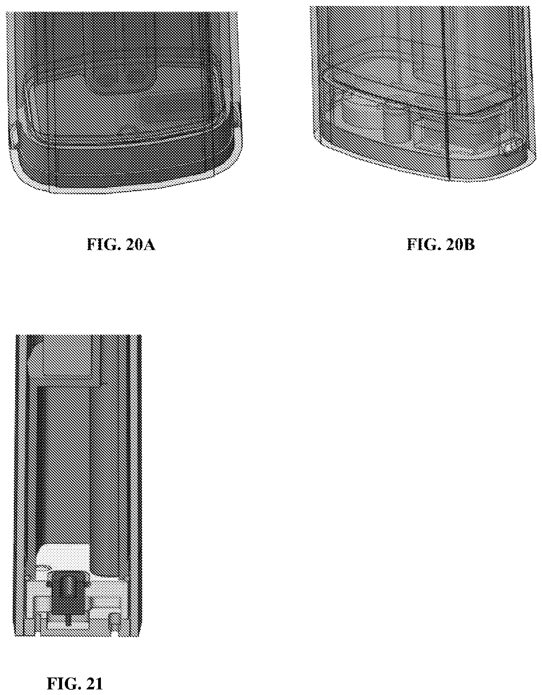

The images in FIGs. 20A and 20B illustrate how a ceramic heating coil can be fit into the device while maintaining the general design and layout of the interior.

The image in FIG. 21 shows a cross section of a ceramic coil embodiment of the device. In this embodiment, the ceramic coil is placed at the bottom of the reservoir and allows the vaporization of the liquid. This embodiment has two air inlets, one towards the left and one towards the right. The size of air inlets may be increased or decreased; it is also possible to only make one air inlet larger and omit the second.

Most prior art devices utilize extra parts to connect the airflow from the mouth piece to the fluid. In addition to the extra cost that these extra parts require, they also add multiple new interfaces that present challenges relating to assembly and leakage.

An alternate design involves molding cavities into the outer shell that can serve as airflow tubes, fluid storage, or battery cavity. The internal construction such as the wick, coil materials, (or ceramic), PCB, battery, and other materials as earlier described, are easily adapted to fit the following constructions as well.

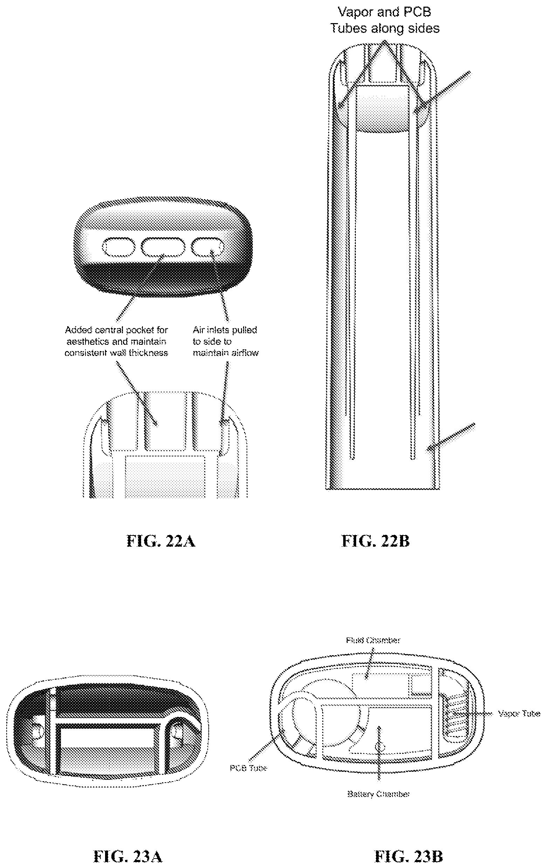

In FIGs. 22A - 24B, it can be seen that the 2 air inlets at the mouth piece are connected to molded cavities inside the outer shell. These cavities are created by molding internal walls. One of the air inlets leads to a pressure sensor that activates the heating element. The second air inlet leads to the wick so that when the heating element is activated, the vapor can travel directly up this tube. In addition to the airflow tubes, additional cavities may be created for fluid storage and the battery. Fluid in the fluid cavity is absorbed by the wick and then evaporated and passed up one of the air cavities. The 4 cavities are sealed off from the external environment and each other with the baseplate. FIGs. 22A - 24B represent a few of many ways to achieve this. Note the images below use multiple air holes at the tapered mouthpiece; this is for illustrative purposes. Either a single air hole, or two or more air holes may be used.

A quartered embodiment uses a molded vapor tube, a molded PCB tube (as earlier described), a molded cavity used for liquid storage, and a molded cavity used for the battery slot. A variety of constructions and placements may be used, depending on the battery size and liquid fill volume that is required.

FIGs. 22A and 22B depict the airflow through vapor and PCB tubes. A similar construction could feasibly also be created by using a single tube for vapor and airflow activation, but these have risks as described above. Furthermore, the aesthetic choices of the airholes and the central aeshtetic pocket may also be varied. For example, aesthetic airholes may be provided that may not necessarily perform a function, but are created for aesthetic symmetry.

FIGs. 23A and 23B depict a fluid chamber, a battery chamber, the PCB tube, and the vapor tube molded into the outer shell to reduce parts required for assembly. It is noteworthy that for some of these constructions to work well, the wick housing may be configured so that the wick may only be in contact with the fluid from one rather than two sides.

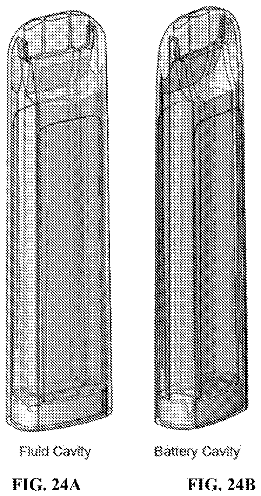

FIGs. 24A and 24B shows how the fluid cavity and the battery cavity can be situated in the design. The left image, labeled as fluid cavity, shows the volume in the interior occupied by the fluid, whereas the right image, shows the volume in the interior occupied by a battery.

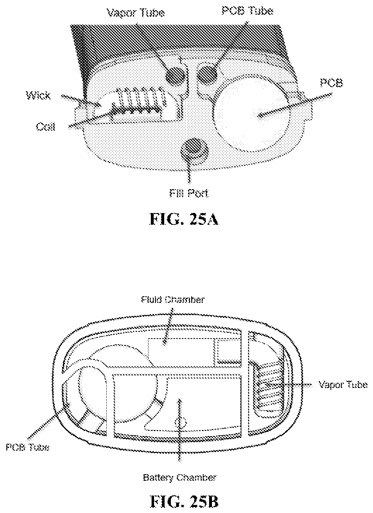

FIG. 25B shows a bottom view and some of the components such as the PCB, Wick, and seals that are substantially equivalent to the constructions in the the Insertable Molded Reservoir shown again in FIG. 25A, but may be optimized to fit the specific layout of the chosen construction; the arrangements of the fill port and the arrangements of the wick/coil/ceramic heating element may vary.

Further, in FIG. 25A, aspects of the airflow path can be observed. The user inhales and through the PCB tube that leads to the the integrated PCB and pressure sensor, a change in pressure is detected. This change in pressure recorded in the pressure sensor triggers the activation of the battery. Subsequently, voltage is delivered to the coil/wick or (ceramic heating element not shown in the embodiment), to start the vaporization process. At the same time, the inhalation of the user through the vapor tube, labeled above, moves air towards the user, and upon the start of the vaporization process, this air is supplemented with an inhalable aerosol generated from heating the liquid with the heating element. The air inlet, not shown above but shown in FIG. 6, serves as an air inlet to the heating chamber. The aerosol generated in the heating chamber then travels throught the vapor tube to the user.

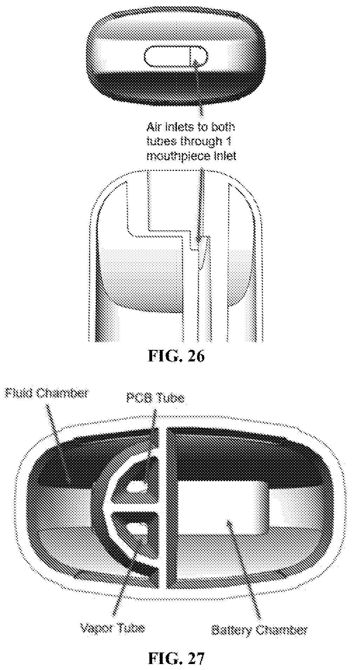

This construction is a further embodiment of molded tubes and a molded reservoir into the device. In this embodiment, the PCB and vapor inlets are on the same side as show in FIG. 16B. As illustrated in FIG. 26, a small barrier is created in this construction should excess liquid travel up the vapor tube, making it more difficult for the liquid to exit the vapor/inhalation hole. Further, a pad of cotton may be fixed to the bottom of the central cavity shown in FIG. 26, further catching excess liquid. Further, the vapor/inhalation hole may be moved further to the left, creating a greater barrier for liquid. FIG. 27 shows the internal construction; the PCB and vapor tubes are centrally anchored, with the fluid chamber on the left and adjacent to the battery chamber on the right.

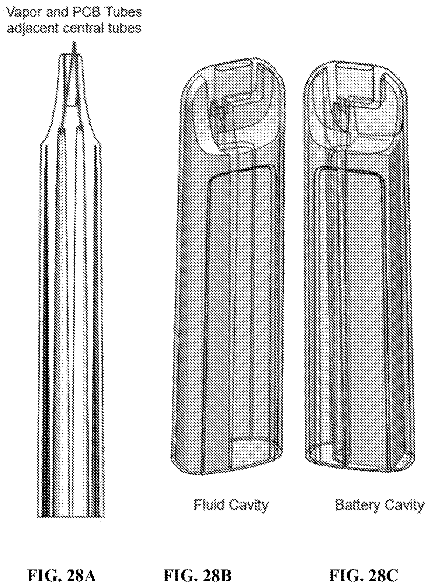

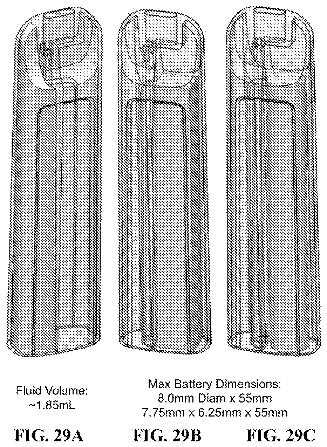

FIGs. 28A - 28B provide a view how the fluid chamber and battery chamber are oriented on the interior of the shell. The fluid cavity and battery cavity may be optimized in size for different fluid capacity and battery capacity requirements. FIGs. 29A - 29C show illustrations of possible battery sizes and fill levels that can fit into the outer shell. The wick configuration, filling port, and sealing are further similar to the other constructions detailed in the invention disclosure above.

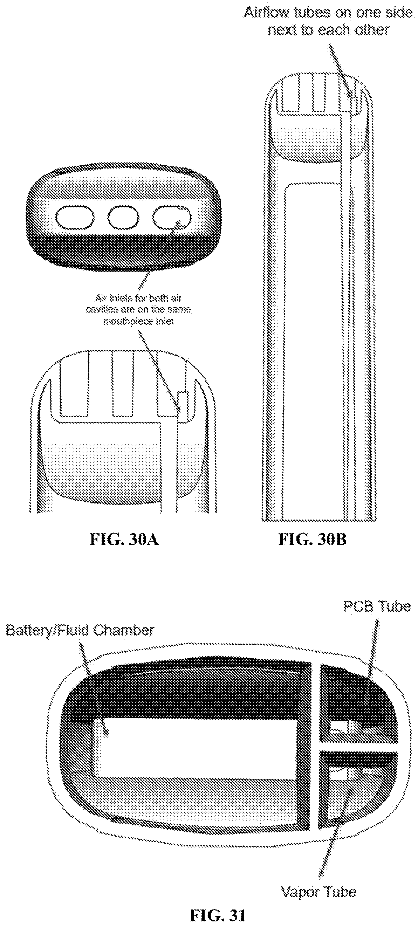

Similar to the Quartered embodiment and the Central Airflow Tubes embodiment, the Side Airflow Tubes embodiment takes advantage of molding components directly into the outer shell. A description of this layout is provided below. FIGs. 30A and 30B show that the airflow and PCB tubes are situated on either the left or the right side, but are molded next to each other. In this embodiment, 2 aesthetic holes are created for symmetry but not for function. However, a central airhole can be used and a barrier for liquid can be created similar to FIG. 26. A cross section view from the bottom is provided in FIG. 31.

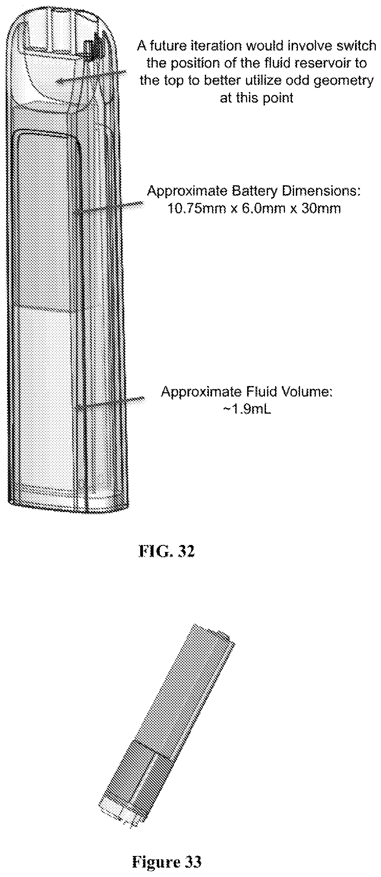

For illustrative purposes, the finished design illustrated positions a separate battery in an upper portion of the interior of the device and the fluid component below. There are a variety of embodiments that may work for this construction, but also, it may be the case that the inserted fluid reservoir and the battery may change place (the fluid reservoir situated above the battery placement). This embodiment would have to take into account how filling is done, and filling may need to get done before the fluid reservoir is inserted. For the illustration, we used the battery on top. FIG. 32 illustrates another construction that places the fluid reservoir on the top to take advantage of the odd geometry; while batteries could not be produced to fit this space, an insertable fluid reservoir could.

FIG. 33 illustrates one approach for doing this using an inserted pod with the battery situated on top/the side as done in the Insertable Molded Fluid Reservoir disclosure. Instead of the vapor and PCB tubes molded into the center of the fluid reservoir, the PCB tube and vapor tube are situated at the side of the shell. The PCB, wick, and filling port remain the same, though their layout may be optimized. FIG. 33 is similar to FIG. 2D, and serves as illustration of how the battery can fit at the top while the inserted reservoir or an inserted pod is fit on the bottom part of the shell.

One of the biggest challenges with molding the outer shell is dealing with the required draft to make the part moldable. Given that the mold tooling must reach very far up into the cavity to create these walls, the effects of draft become quite severe and greatly reduce the amount of space inside the cavity. This problem may be addressed by alternatively using insert molding. Note that this insert molding technique may be applied essentially to all direct-molded constructions detailed in this disclosure.

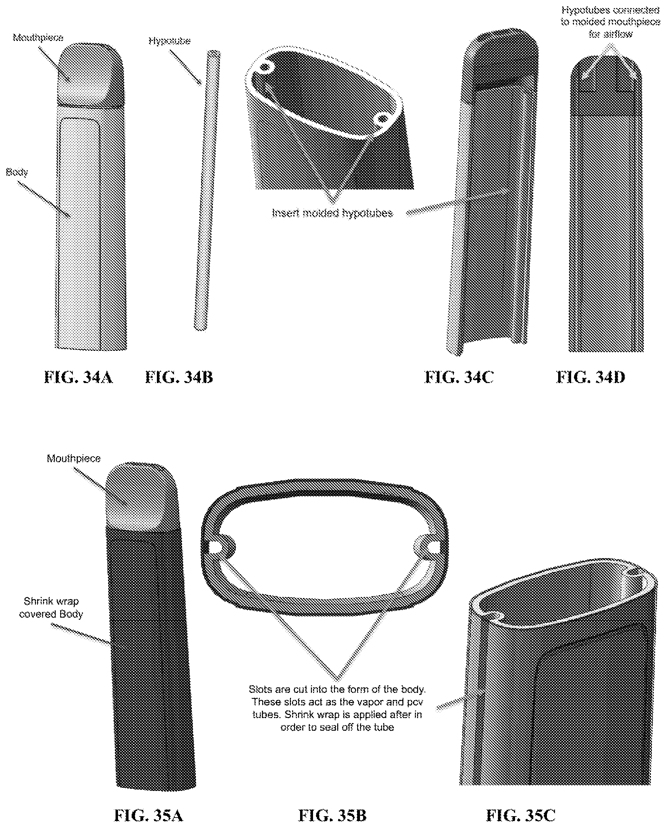

As shown in FIGs. 34A - 34D, instead of creating a cavity using additional walls, a metal hypotube is insert molded into the outer walls of the device. Metal hypotubes can be made from a variety of materials, such as stainless steel 304 or 316. The extruded hypotube does not require draft, and therefore can maintain a consistent wall thickness from top to bottom. This allows more space to be opened up throughout the inner cavity. The tubes are then connected to a molded mouthpiece to allow for airflow through the device. While the mouthpiece shown in FIGs. 34A - 34D appears to be a distinct component from the body; it may either be molded together with the body, or it may be molded separately and attached to the body during assembly.

Another embodiment that avoids the difficulties associated with direct-molded tubes does this by creating a slot in the outer wall of the shell, as illustrated for example in FIGs. 35A -35C. Since this slot is not an additional wall, the draft that is required is the same draft being used for the outer shell to begin with. This allows the slot to be created while minimizing the amount of internal space that it takes up. To then seal off the slot and create an airflow path, heat shrink may be placed over the shell, and heated to shrink down tightly and conform to the shell profile. Since the heat shrink is very thin it adds very little thickness to the device. Examples of industrial shrink wrap materials may include but are not limited to Polyolefin, Polyvinylidene, or a Silicone elastomer. The newly created airflow paths are then connected to paths in the mouthpiece to direct airflow through the slots and to the user.

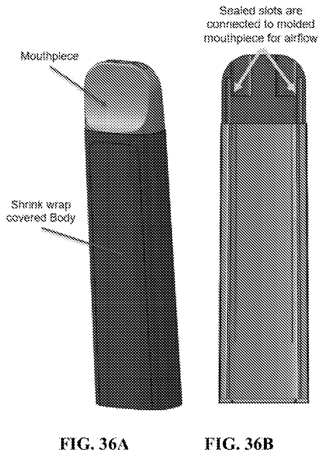

FIGs. 36A and 36B provide and external and an internal view of how industrial shrink-wrapped devices may look, highlighting the airtight tubes created by this sealing process. Battery, reservoir, PCB, wick, can be used as detailed for the other embodiments. The mouthpiece would attach to the main body using either snaps or a press fit. A light seal may be required to maintain an airway, however since we are only sealing against vapor at this point, the contact between the 2 parts may be enough. If a seal is required, a face seal utilizing the compression between the mouthpiece and the body would likely be used.

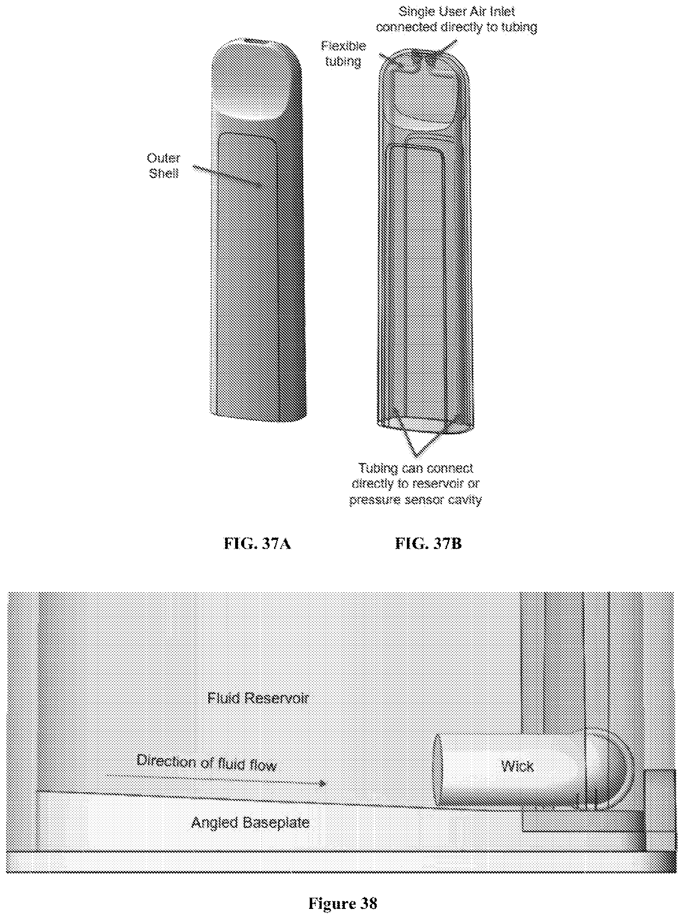

Most components are rigid and are not flexible. When working in tight spaces like the inside of the outer shell, this becomes very difficult. Rigid walls and batteries make it hard to fit components around each other and utilize available space. Another approach consistent with aspects of the present disclosure and illustrated for example by FIGs. 37A and 37B, utilizes flexible tubing in order to gain access to this space. The tubing can be routed around rigid objects such as reservoirs, batteries, and PCBs to make use of the available space. They are connected directly to the air inlet and provide a fluid path to either the pressure sensor or to the heating element, similar as the constructions detailed earlier in this disclosure. For the material of the flexible tubing, materials include but are not limited to silicone tubing.

FIGs 37A and 37B respectively show an exterior and an interior view of an flexible tubing embodiment; battery and fluid reservoir constructions may be assembled in any fashion as detailed by way of example above. The filling port, PCB, wick/coil/ceramic constructions detailed earlier in this disclosure can also be used for this construction.

FIG. 38 illustrates a tilted baseplate feature that enables the device to use as much liquid as possible. Where other devices with a flat plate would have unused fluid leftover at the end, the tilted baseplate works to ensure that all the fluid works its way to the wick. This works because when the user picks up the device to inhale, gravity forces the fluid down the ramp and towards the wick.

It will be understood that, while various aspects of the present disclosure have been illustrated and described by way of example, the invention claimed herein is not limited thereto, but may be otherwise variously embodied within the scope of the following claims..

## Claims

1. A vaporizer device for generating an inhalable aerosol, the device comprising: a body including a mouthpiece and an interior chamber;
a reservoir inserted into the interior chamber of the body adapted for storing an aerosolizable material;
a housing including a heating chamber including a heating element and a pressure sensor configured to control operation of the heating element;
a power source configured to power the heating element and the pressure sensor; and a vapor tube extending from the mouthpiece to the heating chamber; wherein the vapor tube and a PCB tube are integrally formed with the body.

2. The vaporizer device of claim 1, further comprising the PCB tube extending from the mouthpiece to the pressure sensor.

3. The vaporizer device of claim 2, wherein the vapor tube and the PCB tube form respective first and second interior compartments extending longitudinally within the body.

4. The vaporizer device of claim 2, wherein: the power source comprises a battery; and the body further comprises a third interior battery compartment extending longitudinally within the body.

5. The vaporizer device of claim 2, wherein each of the vapor tube and the PCB tube are formed as channels running longitudinally along an exterior surface of the body.

6. The vaporizer of claim 5, further comprising a heat shrink tubing applied over the exterior surface of the body to close the channels.

7. The vaporizer device of claim 1, wherein the power source comprises a battery.

8. The vaporizer device of claim 7, wherein the battery is positioned in the body above the reservoir.

9. The vaporizer device of claim 7, wherein the battery is positioned in the body below the reservoir.

10. The vaporizer device of claim 7, wherein the battery is positioned in the body longitudinally adjacent to the reservoir.

11. The vaporizer device of claim 6, wherein a sealed slot is connected to the mouthpiece to allow airflow to be generated.
